(19) Europäisches Patentamt
European Patent Office
Office européen des brevets

(11) Publication number : 0 126 612 B1

(12) EUROPEAN PATENT SPECIFICATION

(45) Date of publication of patent specification : 19.08.87

(51) Int. Cl.$^4$ : C 07 D207/20, A 61 K 31/40, C 07 D405/04, C 07 D409/04

(21) Application number : 84303277.2

(22) Date of filing : 15.05.84

# (54) 3,4-Dichloro-N-[2-(2,5-dihydro-1H-pyrrol-1-yl)cyclohexyl]-N-methylbenzeneacetamide.

(30) Priority : 18.05.83 US 495857

(43) Date of publication of application : 28.11.84 Bulletin 84/48

(45) Publication of the grant of the patent : 19.08.87 Bulletin 87/34

(84) Designated contracting states : BE CH DE FR GB IT LI NL SE

(56) References cited :
US-A- 4 065 573
US-A- 4 098 904
US-A- 4 145 435
US-A- 4 212 878
US-A- 4 359 476
US-A- 4 360 531
JOURNAL OF MEDICINAL CHEMISTRY, vol. 17, no. 11, November 1974, pages 1188-1193; N.J. HARPER et al.: "1-(3,4-Dichlorobenzamidomethyl)cyclohexyldimethylamine and related compounds as potential analgesics"
The file contains technical information submitted after the application was filed and not included in this specification

(73) Proprietor : THE UPJOHN COMPANY
301 Henrietta Street
Kalamazoo, Michigan 49001 (US)

(72) Inventor : Szmuszkovicz, Jacòb
c/o The Upjohn Company 301 Henrietta Street
Kalamazoo Michigan 49001 (US)
Inventor : McCall, John Michael
c/o The Upjohn Company 301 Henrietta Street
Kalamazoo Michigan 49001 (US)
Inventor : Kaplan, Lester Jay
c/o The Upjohn Company 301 Henrietta Street
Kalamazoo Michigan 49001 (US)
Inventor : McMillan, Moses William
c/o The Upjohn Company 301 Henrietta Street
Kalamazoo Michigan 49001 (US)

(74) Representative : Perry, Robert Edward et al
GILL JENNINGS & EVERY 53-64 Chancery Lane
London WC2A 1HN (GB)

EP 0 126 612 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

A novel compound according to the present invention is an analogue of the given dichloro compounds disclosed in US-A-4 098 904 and US-A-4 145 435. The novel compound is 3,4-dichloro-N-[2-(2,5-dihydro-1H-pyrrol-1-yl) cyclohexyl]-N-methyl-benzeneacetamide which has the formula

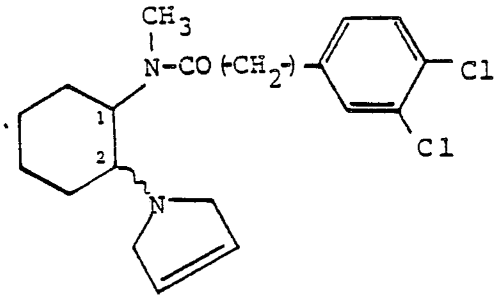

(I)

and the invention relates also to its pharmacologically-acceptable acid addition salts. The novel compound and its salts may be formulated for therapeutic use, in dosage unit form.

The novel compound (I) and its salts in their crystalline state may sometimes be isolated from their reaction mixtures as solvates, i. e. with a discrete quantity of solvent, e. g. water, ethyl acetate, methanol or methylene chloride, associated physically, and thus not affecting the chemical entity per se.

It will be recognised by those skilled in the organic chemical art that the carbon atoms at positions 1 and 2 of the cycloaliphatic ring of structure (I) to which nitrogens are bonded are asymmetrically substituted. Each of these carbon atoms can independently possess and R or S-configuration and thus a compound of the formula (I) has 4 stereoisomers which comprise two pairs of enantiomers ; each enantiomeric pair is termed a racemate. The racemates respectively have the nitrogen-containing groups at positions 1 and 2 of structure (I) in a trans or cis orientation, i. e. the groups are on opposite sides of the plane of the cycloaliphatic ring or on the same side of the cycloaliphatic ring. The racemates of structure (I) compounds can each exist as a mixture of the two enantiomers, or each enantiomer of each pair can be separated. Varying mixtures of enantiomers are also possible. When it is desired to specify the configuration of the other asymmetric centre relative to that of position 1, this is done according to the Chemical Abstracts Service publication, « Naming and Indexing of Chemical Substances for Chemical Abstracts during the Ninth Collective Period (1972-1976) », a reprint of Section IV (Selection of Index Names for Chemical Substances) from the Chemical Abstracts Volume 76 Index Guide. Accordingly, the relative stereochemistry of the asymmetric carbon atoms is indicated by the arbitrary designation of 1α for the orientation of the substituent at C-1 and the designation 2α or 2β when the substituent at C-2 is on the same or opposite side of the plane of the cycloaliphatic ring, respectively, relative to the C-1 substituent.

If desired, the formula I compound can be resolved into its respective d- and l-optical isomers by methods known in the art. The optical resolution can be done by at least two different routes. The resolving agent by either route is any of the known resolving agents, such as optically-active camphorsulfonic acid, bis-o-toluoyltartaric acid, tartaric acid or diacetyltartaric acid, which are commercially-available and which are commonly used for resolution of amines (bases), as for example in Organic Synthesis, Coll. Vol. V, p. 932 (1973), resolution of R-(+) and S-(—)-α-phenylethylamine with (—)-tartaric acid.

By the first method for resolving the compound of this invention, for example, it is converted into its optically-active diastereomeric salts by reaction with an optically-active acid in a manner standard in the isomer resolution art. These diastereomeric salts can then be separated by conventional means such as differential crystallisation. Diastereomeric salts have different crystallisation properties, which are taken advantage of in this separation. On neutralisation of each diastereomeric salt with aqueous base, the corresponding optically-active enantiomers of the free amino-amide can be obtained, each of which can subsequently and separately be converted as hereinafter described in the examples to the desired acid addition salt.

By the second method, the diamine 1-methylamino-2-(2,5-dihydro-1H-pyrrol-yl) cyclohexane is resolved into its d- or l-isomer by treatment with the resolving agent, crystallisation, separation and regeneration of the respective d- and l-compounds, for example, trans-d-diamine, trans-l-diamine, or the cis-d-diamine and cis-l-diamine, and the resolved diamine is then reacted with an aracyl compound selected from N-(3,4-dichlorophenyl) imidazole, 3,4-dichlorobenzoyl halides and 3,4-dichlorobenzoic acid in the presence of a condensing agent to form the respective cis or trans-d- or l-compound of formula I, which can then be converted to any desired pharmaceutically-acceptable acid addition salt by procedures exemplified hereinafter.

In general, the compound of formula I can be prepared by reacting the given diamine (1) with the given aracyl imidazole ; (2) with the given aracyl chloride or bromide in the presence of an acid scavenger

such as triethylamine ; or (3) with the given benzoic carboxylic acid in the presence of a condensing agent, such as a carbodiimide, in an organic solvent for the reactants, preferably in a chlorinated alkane, e. g., methylene chloride, ethylene dichloride, chloroform or carbon tetrachloride or in an ether solvent such as diethyl ether, or a cyclic ether solvent such as tetrahydrofuran (THF) or dioxane, until the compound of this invention is produced. Carbodiimides such as dicyclohexylcarbodiimide or diisopropylcarbodiimide can be used as condensing agents.

The reactants can be mixed in substantially equimolar proportions to effect formation of the desired product (I), but in cases where the non-pertinent amino nitrogens are protected against reaction, if one of the reactants is more expensive than the other, it is sometimes preferred to use a stoichiometric excess of the less expensive reactant to insure that substantially all of the more expensive reactant is consumed in the reactions. The reaction will proceed at ambient temperature for most combinations of reactants, but for some combinations of reactants, variations from the initial to final reaction conditions may vary between — 25 °C and reflux temperature of the mixture depending on the reactivity of the reactants, the desired reaction time, the solvent being used, the molar proportions, and similar factors of concern to the chemist operating the process.

Procedures for preparing the aracyl imidazoles and aracyl halide reactants used to form compounds of this invention are known in the art. See, for example, R. B. Wagner and H. D. Zook, Synthetic Organic Chemistry, 1953, John Wiley and Sons, Chapter 17, p. 546 et seq. The aracyl imidazole can be prepared in situ by reacting carbonyldiimidazole with the given benzoic acid in an organic solvent. The given benzoic acid is either known in the art or is prepared by methods known in the art.

Acid addition salts can be prepared by reacting a formula I free base with a stoichiometric amount of an acid, such as hydrogen chloride, hydrogen bromide, hydrogen iodide, sulfuric acid, phosphoric acid, acetic acid, lactic acid, citric acid, succinic acid, benzoic acid, salicyclic acid, pamoic acid, cyclohexanesulfamic acid, methanesulfonic, naphthalenesulfonic, p-toluenesulfonic, maleic, furmaric or oxalic acids. The reaction can be carried out in aqueous or organic liquid solvent or non-aqueous media such as diethyl ether or ethyl acetate. Non-aqueous media are preferred. When it is desired to obtain optically resolved products in crystalline form, it may be more convenient to form salts such as maleates, citrates or pamoates rather than the inorganic acid addition salts, such as the hydrochlorides. Also, whereas oxalic acid and other equivalent acids can be used to produce the product in a more easily handled solid form, e. g., in plant manufacturing isolation procedures, it would preferably not be used as a pharmaceutically acceptable salt form of the amino-amide product.

In preparing the compounds of this invention the source of the 2,5-dihydro-1H-pyrrol-1-yl moiety is generally 2,5-dihydro-1H-pyrrole (also known as 3-pyrroline) which is known in the literature (Chemical Abstracts Registry Number 109-96-6) and is presently commercially available.

Procedures for preparing the diamine starting materials have already been described in US-A-4 098 904, US-A-4 145 435, US-A-4 359 476, US-A-4 360 531, DE-A-3 241 193 and BE-A-0 895 002, it being necessary only to substitute the 2,5-dihydro-1H-pyrrole for the amine groups listed for placement in the 2-position of the cycloalkyl rings of the diamines listed therein, recognizing that reaction conditions chosen should not unfavorably alter the double bond of the 2,5-dihydro-1H-pyrrole ring.

The term « dosage unit form » as used in this specification refers to physically discrete units suitable as unitary dosages for mammalian subjects, each unit containing as the essential active ingredient a predetermined quantity of a compound of this invention with the required pharmaceutical means which adapt said ingredient for systemic administration. The specification for the novel dosage unit forms of this invention are dictated by and directly dependent on the physical characteristics of the essential active ingredient and the particular effect to be achieved in view of the limitations inherent in the art of compounding such an essential active material for beneficial effects in humans and animals, these being features of the present invention. Examples of suitable dosage unit forms in accordance with this invention are tablets, capsules, orally administered liquid preparations in suitable liquid vehicles, sterile preparations in suitable liquid vehicles for intramuscular and intravenous administration, suppositories and sterile dry preparations for the extemporaneous preparation of sterile injectable preparations in a suitable liquid vehicle. Suitable solid diluents or carriers for the solid oral pharmaceutical dosage unit forms are selected from the group consisting of lipids, carbohydrates, proteins and mineral solids, for example, starch, sucrose, lactose, kaolin, dicalcium phosphate, gelatin, acacia, corn syrup, corn starch, talc and the like. Capsules, both hard and soft, are filled with compositions of these amino-amide active ingredients in combinations with suitable diluents and excipients, for example, edible oils, talc, calcium carbonate and the like and also calcium stearate. Liquid preparations for oral administration are prepared in water or aqueous vehicles which advantageously contain suspending agents, for example, methylcellulose, acacia, polyvinylpyrrolidone, polyvinyl alcohol and the like. In the case of injectable forms, the injectable formulation must be sterile and must be fluid to the extent that easy springeability exists. Such preparations must be stable under the conditions of manufacture and storage, and ordinarily contain in addition to the basic solvent or suspending liquid, preservatives in the nature of bacteriostatic and fungistatic agents, for example, parabens, chlorubutanol, benzyl alcohol, phenol, thimerosal, and the like. In many cases, it is preferable to include osmotically active agents, for example, sugars or sodium chloride in isotonic concentrations. Carriers and vehicles include vegetable oils, ethanol, polyols, for example, glycerol, propylene glycol, liquid polyethylene glycol, and the like. Any solid preparations for

subsequent .extemporaneous preparation of sterile injectable preparations are sterilized, preferably by exposure to a sterilizing gas, for example, ethylene oxide. The aforesaid carriers, vehicles, diluents, excipients, preservatives, isotonic agents and the like constitute the pharmaceutical means which adapt the preparations for systemic administration.

The pharmaceutical dosage unit forms are prepared in accordance with the preceding general description to provide from about 0.5 to about 350 mg of the essential active ingredient per dosage unit form, which as aforesaid may be in the form of a semi-solid or solid, topical, oral or rectal preparation, a liquid oral preparation, an injectable preparation including liquid preparations and solid dry preparations for extemporaneous reconstitution to a liquid injectable preparation. The amount of the essential active ingredient provided in the pharmaceutical dosage unit forms is that amount sufficient to obtain analgesic effects within the aforesaid effective non-toxic range. Expressed otherwise, when used systemically, an amount of the essential active ingredient is provided to a recipient within a range from about 0.01 mg per kg to about 5 mg per kg of body weight of the recipient. Preferred dosages for most applications are 0.05 to 2.0 mg per kg of body weight.

The useful pharmaceutical dosage unit forms of these compounds in pharmaceutical formulations are preferably adapted for systemic administration to obtain analgesic effects comprising an effective, non-toxic amount of a compound according to formula I or as its pharmacologically-acceptable salt.

The novel compound has the advantage of having lower physical dependence liability than known analgesic compounds such as morphine and methadone, as shown by evaluation of representative compounds and those standard analgesic drug compounds in various pharmacological test procedures which measure analgesia and the physical dependence liability of the test compounds in standard laboratory test animals.

The formula I compound has an $ED_{50}$ value of less than 10 mg/kg s. c. (subcutaneous administration) in standard laboratory animal analgesic tests such as the tail flick, pinch, and hydrochloric acid writhing tests, while at the same time possessing low apparent physical dependence liability as compated to commercial analgesics used as standards. The procedures used to determine these properties are essentially those of Way et al., (Way, E.L. et al., « Simultaneous Quantitative Assessment of Morphine Tolerance and Physical Dependence «, J. Pharmacol. Exp. Ther., 167, pp. 1-8 (1969)) and Saalens et al., (Saalens, J. K. et al., « The Mouse Jumping Test — A Simple Screening Method to Estimate the Physical Dependence Capacity of Analgesics », Arch. Int. Pharmacodyn., 190, pp. 213-218 (1971)). Statistical effective doses ($ED_{50}$ values) and 95 % confidence limits were calculated by the method of Spearman and Karber (Finney, D.J., « Statistical Methods in Biological Assay », Hafner Publ., (1952)).

Known analgesic drugs such as morphine and methadone exhibit analgesic $ED_{50}$ values of less than 2 mg/kg s. c., respectively, in these standard analgesic tail flick, pinch and writhing tests, but are known to have high apparent physical dependence liability effects, and this is confirmed by their (morphine and methadone) having relatively low naloxone jumping $ED_{50}$ values ranging from 12 to 30 mg/kg s. c.

(1α,2β)-(±)-3,4-Dichloro-N-[2-(2,5-dihydro-1H-pyrrol-1-yl) cyclohexyl]-N-methylbenzeneacetamide (see Example 1 below) is surprisingly and unexpectedly more potent in these analgesic tail flick, pinch and writhing tests than the known compound, (1α,2β)-(±)-3,4-dichloro-N-methyl-N-[2-(1-pyrrolidinyl) cyclohexyl] benzeneacetamide (the compound of Example 14 of U.S. Patent 4,145,435, listed above). It has further been found that the (—)-enantiomer form (see Example 2 below) of this representative compound of this invention is more potent in these same analgesic tests than either the racemate form (Example 1 below) or the (+)-enantiomer form (see Example 3 below) of this compound of this invention has surprising potency in these same analgesic tests.

The invention is further exemplified by the following detailed examples, the procedures of which can be used to prepare compounds of this invention.

Example 1

(1α,2β)-(±)-3,4-Dichloro-N-[2-(2-,5-dihydro-1H-pyrrol-1yl) cyclohexyl]-N-methylbenzeneacetamide and its maleate salt

A. Preparation of (1α,2β)-(±)-2-(2,5-dihydro-1H-pyrrol-1-yl)-N-methylcyclohexanamine

Cyclohexene epoxide (32.67 g., 0.333 mole) and 2,5-dihydro-1H-pyrrole (23.6 g., 0.333 mole) in 60 ml of water are heated for 24 hours at 70 °C. The mixture is partitioned between methylene chloride and water. The organic phase is dried over sodium sulfate and concentrated in vacuo. The residue is distilled (80°-90 °C/0,4 mm Hg) (53.3 Pa units) to yield 43.36 g of (1α,2β)-2-(2,5-dihydro-1H-pyrrol-1-yl) cyclohexanol as an oily liquid. All of this is dissolved in 600 ml of methylene chloride and stirred at 0 °C with 32 g (0.31 mole) of triethylamine. Methanesulfonyl chloride (35.51 g., 0.31 mole) is added and the mixture is stirred for 35 min. at 0 °C. The solution is partitioned with ice water. The organic phase is dried over sodium sulfate and concentrated in vacuo. The residue is transferred to a 1-liter Parr Pressure Reactor. A solution of 40 % methylamine in water (150 ml) is added and the mixture is heated at 70 °C for 20 hours. The mixture is then partitioned with diethyl ether and 10 % aqueous sodium hydroxide. The ether layer is extracted with 1.2 M HCl. The acidic aqueous layer is extracted with diethyl ether. The acidic aqueous

layer is then made basic with 10 % sodium hydroxide and extracted with diethyl ether. The ether layer is washed with brine and concentrated. The residue is dissolved in methylene chloride, dried over sodium sulfate, and concentrated in vacuo. The residue is distilled (80 °C/0.3 mm Hg) (39.99 Pa units) to yield 39.0 g of (1α,2β)-(±)-2-(2,5-dihydro-1H-pyrrol-1-yl)-N-methylcyclohexanamine.

B. (1α,2β)-(±)-3,4-Dichloro-N-[2,5-dihydro-1H-pyrrol-1-yl) cyclohexyl]-N-methylbenzeneacetamide and its maleate salt

A mixture of 4.41 g (24.5 mmole) of the (1α,2β)-(±)-2-(2,5-dihydro-1H-pyrrol-1-yl)-N-methylcyclohexanamine from Part A above, 5.16 g (31.8 mmole) of N,N'-carbonyldiimidazole and 6.52 g (31.8 mmole) of 3,4-dichlorophenylacetic acid in 100 ml of methylene chloride is stirred for 20 hours. The mixture is washed with aqueous sodium bicarbonate solution ; the organic layer is dried over anhydrous sodium sulfate, filtered and concentrated in vacuo. The residue is chromatographed on silica gel eluting with 2 % (10 % concentrated ammonium hydroxide in methanol)-98 % ethyl acetate to give 2.10 g of the titled amino-amide. This product is partitioned between methylene chloride and water, and the organic layer is dried over anhydrous sodium sulfate, filtered, and concentrated in vacuo. This titled amino-amide is converted to its maleate salt by reaction with an equivalent amount of maleic acid in diethyl ether solution to give, after evaporation and trituration with ethyl acetate, 1.83 g of the titled amino-amide maleate, mp 200-202 °C. The nmr and mass spectra support the titled amino-amide structure. This product has a C : H : N ratio of 57.21 : — 5.90 :.5.58 ; calculated for $C_{19}H_{24}N_2Cl_2O \cdot C_4H_4O_4$ : 57.14 : — 5.84 : 5.97.

Repetition of this experiment on a two-fold larger scale gives 7.98 g of the titled amino-amide after chromatography, and 7.58 g of the titled amino-amide after partitioning this material with aqueous sodium bicarbonate, drying over anhydrous sodium sulfate, filtering and concentrating in vacuo.

## Example 2

[1S-(1α,2β)]-(—)-3,4-dichloro-N-[2-(2,5-dihydro-1H-pyrrol-1-yl)-cyclohexyl]-N-methylbenzeneacetamide and its maleate salt

A. Resolution of (1α,2β)-(±)-2-(2,5-dihydro-1H-pyrrol-1-yl)-N-methylcyclohexanamine. Preparation of (1α,2β)-(+)-2-(2,5-dihydro-1H-pyrrol-1-yl)-N-methylcyclohexanamine.

3.64 g (20.2 mmole) of the subtitled racemic amine and 7.81 g (20.2 mmole) of di-p-toluoyl-d-tartaric acid are each separately dissolved in a minimum volume of methanol, and the two solutions are mixed together.

Crystallization starts very quickly. After 2 days of standing the mixture is filtered. There is obtained 8.00 g of a white crystalline solid (A). The solid is recrystallized from 300 ml of hot (60 °C) methanol to obtain 5.00 g of crystalline material (B), m. p. 186-188 °C. This crystalline material is recrystallized from 100 ml of methanol to obtain 3.27 g (C) of white crystalline material, m. p. 187°-188 °C.

Small samples of crystalline materials (B) and (C) above are partitioned between ethyl acetate and 10 % (w/v) sodium hydroxide aqueous solution. There is obtained 39 mg of crystalline material from sample B which was dissolved in 4.0 ml of methanol. The $[\alpha]_\lambda^{25\,°C}$ rotation constants for this sample are :

| λ | Hg 578 | 546 | 436 | 365 | Na 589 |
|---|---|---|---|---|---|
| $[\alpha]_\lambda^{25°C}$ | 114.8° | 129.8° | 216.1° | 328.6° | 110° |

From Sample C, there is obtained 21 mg of crystalline material which is dissolved in 2.0 ml of methanol. The $[\alpha]\lambda^{25\,°C}$ rotation figures are :

| λ | Hg 578 | 546 | 436 | 365 | Na 589 |
|---|---|---|---|---|---|
| $[\alpha]_\lambda^{25°C}$ | 114.6° | 129.6° | 215.6° | 327.8° | 109.8° |

The remainder of the 3.27 g portion of the crystalline material (C) is partitioned between 10 percent w/v sodium hydroxide aqueous solution and ethyl acetate ; the organic layer is washed twice with brine and is dried over sodium sulfate. The solvent is evaporated under vacuum, the residue is dissolved in methylene chloride, dried with sodium sulfate and the solvent is evaporated under vacuum to obtain 1.20 g (D) of the (1α,2β)-(+)-2-(2,5-dihydro-1H-pyrrol-1-yl)-N-methylcyclohexanamine. A 39 mg portion of this material (D) is dissolved in 4.0 ml of methanol and the $[\alpha]\lambda^{25\,°C}$ rotation figures are measure as above.

| λ | Hg 578 | 546 | 436 | 365 | Na 589 |
|---|---|---|---|---|---|
| $[\alpha]_\lambda^{25°C}$ | +104.2° | +117.7 | +196 | +297.6° | +99.89° |

B. [1S-(1α,2β)]-(—)-3,4-Dichloro-N-methyl-N-[2-(2,5-dihydro-1H-pyrrol-1-yl) cyclohexyl] benzeneacetamide, and its maleate salt.

A mixture of 1.20 g (6.67 mmole) of the (+)-diamine from Part A above, 1.30 g (8.00 mmole) of N,N'-carbonyldiimidazole and 1.64 g of 3,4-dichlorophenylacetic acid in 25 ml of methylene chloride is stirred overnight and purified as described in Example 1, Part B above to give 1.30 g of the pure titled (—)-amino-amide. The Nuclear Magnetic Resonance spectrum of the product is in excellent agreement with this named compound. The $[\alpha]\lambda^{25\,°C}$ rotation figures, obtained by dissolving a 14 mg sample of the product in 1.50 ml of methanol are :

| λ | Hg 578 | 546 | 436 | 365 | Na 589 |
|---|---|---|---|---|---|
| $[\alpha]\lambda^{25°C}$ | -10.5° | -12.4° | -24.8° | -47.6° | -10.0° |

The maleate salt of this (—)-amino-amide compound is prepared by adding a mixture of 0.96 g of the above titled (—)-amino-amide free base (2.6 mmole) to 0.35 g of maleic acid in ethyl acetate at 60 °C. The solvent is removed. The residue is triturated with ethyl acetate to obtain 660 mg of the titled maleate salt as a white solid, mp 168-169 °C. A 10 mg portion of this white crystalline solid is dissolved in 1.1 ml of methanol (concentration 9.09 mg/ml) and the $[\alpha]\lambda^{25\,°C}$ rotation figures are measured on this solution :

| λ | Hg 578 | 546 | 436 | 365 | Na 589 |
|---|---|---|---|---|---|
| $[\alpha]\lambda^{25°C}$ | -20.5° | -23.6° | -44.2° | -44.2° | -19.5° |

The analytical sample from the 660 mg lot has a C : H : N ratio of 57.33 : 5.89 : 5.72. Calculated for $C_{19}H_{24}N_2Cl_2O \cdot C_4H_4O_4$ : 57.14 : 5.84 : 5.97.

A second crop (80 mg) of maleate salt from the mother liquors from the trituration has a melting point of 169-170 °C.

The absolute configuration of the titled (—)-amino-amide is determined by hydrogenation to the known [1S-(1α,2β)]-(—)-3,4-dichloro-N-methyl-N-[2-(1-pyrrolidinyl) cyclohexyl] benzeneacetamide which can also be named trans-(1)-2-(3,4-dichlorophenyl)-N-methyl-N-[2-(1-pyrrolidinyl) cyclohexyl] acetamide, the compound of Example 35 of Szmuszkovicz U.S. Patent 4,145,435.

Thus a solution of 50 mg of the titled (—)-amino-amide in 25 ml of ethyl acetate is hydrogenated for 1.5 hours at atmospheric pressure over platinum (from 15 mg of platinum oxide). The solution was vigorously magnetically stirred. The platinum was removed by filtering through Celite® filter aid, and the Celite® was washed with two 5 ml portions of ethyl acetate. Evaporation of the solvent afforded the known [1S-(1α,2β)]-(—)-3,4-dichloro-N-methyl-N-[2-(1-pyrrolidinyl) cyclohexyl] benzeneacetamide. The titled (—)-amino-amide thus has the (1S,2S) absolute configuration.

## Example 3

[1R-(1α,2β)]-(+)-3,4-dichloro-N-[2-(2,5-dihydro-1H-pyrrol-1-yl)-cyclohexyl]-N-methylbenzeneacetamide and its maleate salt

A. Resolution of (1α,2β)-(±)-2-(2,5-dihydro-1H-pyrrol-1-yl)-N-methyl-cyclohexanamine. Preparation of (1α,2β)-(—)-2-(2,5-dihydro-1H-pyrrol-1-yl)-N-methylcyclohexanamine.

1.77 g (9.83 mmole) of the subtitled racemic diamine and 3.97 g (10.3 mmole) of di-p-toluoyl-1-tartaric acid are each separately dissolved in a minimum volume of methanol and the two solutions are mixed together. Crystallization occurs and the solid is filtered and recrystallized from 100 ml of methanol. The resulting solid is partitioned between ethyl acetate and 10 % (w/v) sodium hydroxide aqueous solution. The organic layer is washed with brine, dried over anhydrous magnesium sulfate, and concentrated in vacuo to give 0.46 g of the subtitled (—)-diamine. A 54 mg sample of this (—)-diamine is dissolved in 5.0 ml of methanol (concentration 10.8 mg/ml) and the rotation figures are measured as above :

| λ | Hg 578 | 546 | 436 | 365 | Na 589 |
|---|---|---|---|---|---|
| $[\alpha]\lambda^{25°C}$ | -102.6° | -116.2° | -192.9° | -293.2° | -98.5° |

B. [1R-(1α,2β)]-(+)-3,4-dichloro-N-[2-(2,5-dihydro-1H-pyrrol-1-yl)-cyclohexyl]-N-methylbenzeneacetamide, and its maleate salt.

A mixture of 0.73 g (3.6 mmole) of 3,4-dichlorophenylacetic acid, 0.46 g (2.6 mmole) of the (—)-diamine from Part A above and 0.58 g (3.6 mmole) of N,N'-carbonyldiimidazole in 25 ml of methylene

chloride is stirred overnight and purified as described in Example 1, Part B above to give 0.57 g of the pure titled (+)-amino-amide product. The maleate salt of this 0.57 g of titled (+)-amino-amide is prepared by reaction with 0.21 g of maleic acid. Upon recrystallizing this maleate salt from ethyl acetate, the first crop (50 mg) has a melting point of 183-186 °C. A 12 mg portion of this maleate salt was dissolved in 1 ml of methanol. The $[\alpha]\lambda^{25\,°C}$ rotations were :

| Hg 578 | 546 | 436 | 365 | Na 589 |
|---|---|---|---|---|
| +8.5° | 9.9° | 18.5° | 34° | 8.2 |

The residue was recrystallized from a 50 : 50 v/v methylene chloride-diethyl ether mixture to obtain oily crystals which were triturated with diethyl ether to obtain 330 mg of the titled (+)-amino-amide maleate salt as an off-white solid which softened at 123 °C (no clear melting point) and which has the following rotations (11.5 mg/ml is methanol) :

| λ | Hg 578 | 546 | 436 | 365 | Na 589 |
|---|---|---|---|---|---|
| $[\alpha]\lambda^{25°C}$ | +19.6° | +22.8° | +43.8° | +82.3 | +18.8 |

**Claims**

1. 3,4-Dichloro-N-[2-(2,5-dihydro-1H-pyrrol-1-yl) cyclohexyl]-N-methylbenzeneacetamide or a pharmacologically-acceptable acid addition salt thereof.

2. (1α,2β)-(±)-3,4-Dichloro-N-[2-(2,5-dihydro-1H-pyrrol-1-yl) cyclohexyl]-N-methylbenzeneacetamide or its maleate salt.

3. [1S-(1α,2β)]-(—)-3,4-Dichloro-N-[2-(2,5-dihydro-1H-pyrrol-1-yl) cyclohexyl]-N-methylbenzeneacetamide or its maleate salt.

4. [1R-(1α,2β)]-(+)-3,4-Dichloro-N-[2-(2,5-dihydro-1H-pyrrol-1-yl) cyclohexyl]-N-methylbenzeneacetamide or its maleate salt.

5. A compound according to any preceding claim, for therapeutic use.

**Patentansprüche**

1. 3,4-Dichloro-N-(2-(2,5-Dihydro-1H-Pyrrol-1-yl) Cyclohexyl)-N-Methylbenzolacetamid oder ein pharmakologisch akzeptables saures Zusatzsalz davon.

2. (1α,2β)-(±)-3,4-Dichloro-N-(2-(2,5-Dihydro-1H-Pyrrol-1-yl) Cyclohexyl)-N-Methylbenzolacetamid oder dessen Maleatsalz.

3. (1S-(1α,2β)-(—)-3,4-Dichloro-N-(2-(2,5-Dihydro-1H-Pyrrol-1-yl) Cyclohexyl)-N-Methylbenzolacetamid oder dessen Maleatsalz.

4. (1R-(1α,2β)-(+)-3,4-Dichloro-N-(2-(2,5-Dihydro-1H-Pyrrol-1-yl) Cyclohexyl)-N-Methylbenzolacetamid oder dessen Maleatsalz.

5. Eine Verbindung nach einem der vorhergehenden Ansprüche für therapeutische Zwecke.

**Revendications**

1. 3,4-dichloro-N-[2-(2,5-dihydro-1H-pyrrol-1-yl) cyclohexyl]-N-méthylbenzène-acétamide ou un de ses sels d'addition d'acides pharmacologiquement acceptables.

2. (1α,2β)-(±)-3,4-dichloro-N-[2-(2,5-dihydro-1H-pyrrol-1-yl) cyclohexyl]-N-méthylbenzène-acétamide ou son maléate.

3. [1S-(1α,2β)]-(—)-3,4-dichloro-N-[2-(2,5-dihydro-1H-pyrrol-1-yl) cyclohexyl]-N-méthylbenzène-acétamide ou son maléate.

4. [1R-(1α,2β)]-(+)-3,4-dichloro-N-[2,5-dihydro-1H-pyrrol-1-yl) cyclohexyl]-N-méthylbenzène-acétamide ou son maléate.

5. Composé suivant l'une quelconque des revendications précédentes, destiné à une utilisation thérapeutique.